# EUROPEAN PATENT APPLICATION

(11) **EP 3 238 688 A1**
(43) Date of publication of application: **01.11.2017**
(21) Application number: 17166843.7
(22) Date of filing: 18.04.2017
(51) Int. Cl.: A61H 23/00, A61H 9/00, A61H 23/02

(54) **MASSAGE MACHINE**

(30) Priority: 25.04.2016 JP 2016086827
(71) Applicant: Family Inada Co., Ltd., Osaka 532-0004 (JP)
(72) Inventor: YUJI, Notsu, Saihaku-gun, Tottori 689-3224 (JP); YUTA, Ishidou, Saihaku-gun, Tottori 689-3224 (JP); TOMOHARU, Fukuda, Saihaku-gun, Tottori 689-3224 (JP); ATSUSHI, Moritomo, Saihaku-gun, Tottori 689-3224 (JP)
(74) Representative: Horn Kleimann Waitzhofer Patentanwälte PartG mbB

(57) **Abstract**

An object is to provide a massage machine which is able to perform a massage tuned to the tempo of a musical piece. Provided is a massage machine including a massage portion 5 that performs a massage with respect to a user, and a control unit 6 that controls an operation of the massage portion 5. One or a plurality of first massage courses in which the massage portion 5 performs an operation determined in advance in a manner corresponding to one or a plurality of particular musical pieces A to D are stored. The control unit 6 is able to execute the first massage course. The massage machine includes first musical piece reproduction equipment 21 that is able to acoustically reproduce an acoustic signal included in the musical pieces A to D, and a storage section 30 that stores the one or the plurality of particular musical pieces A to D. The first musical piece reproduction equipment 21 is controlled by the control unit 6.

## Description

### TECHNICAL FIELD

The present invention relates to a massage machine.

### BACKGROUND ART

In the related art, in massage machines provided with an air bag, there is a known massage machine in which a control signal is generated based on an acoustic signal input from a sound source, and an operation of an air supply/exhaust device is controlled in response to the generated control signal (for example, refer to Patent Document 1). The massage machine generates the control signal for causing the air supply/exhaust device to perform air supply or exhaust with respect to the air bag in accordance with timing the input acoustic signal reaches a predetermined threshold value.

### RELATED ART DOCUMENT

### PATENT DOCUMENT

Patent Document 1: JP-A-2003-079687

### SUMMARY OF THE INVENTION

### PROBLEM THAT THE INVENTION IS TO SOLVE

Incidentally, in a drive system of a massage portion including a motor and an air supply/exhaust device, there exists a response lag with respect to a control signal. For example, in a massage portion having a treatment member of which the drive system is a motor, due to a transmission mechanism such as a speed reducer interposed between the motor and the treatment member, or inertia of the motor, a lag is caused in an operation of the treatment member with respect to an acoustical output of an acoustic signal. In addition, in a massage portion having an air bag of which the drive system is an air supply/exhaust device, due to an air hose interposed between the air supply/exhaust device and the air bag, a lag is caused in an operation of the air bag with respect to an acoustical output of an acoustic signal. Therefore, the present invention has been made in order to solve the aforementioned problems, and an object thereof is to provide a massage machine which is able to perform a massage tuned to the tempo of a musical piece.

### MEANS FOR SOLVING THE PROBLEM

The present invention includes a massage portion that performs a massage with respect to a user, and a control unit that controls an operation of the massage portion. One or a plurality of first musical pieces in which the massage portion performs an operation determined in advance in a manner corresponding to one or a plurality of particular massage courses are stored. The control unit is able to execute the first massage course.

According to the configuration, the massage courses tuned to tempos of the particular musical pieces designated by a manufacturer are designed in advance. Therefore, there is no need for the operation of the massage portion to successively correspond to a variation of the acoustic signal, and it is possible to perform a massage intended by the manufacturer so as to be tuned to the tempos of the musical pieces.

In addition, it is preferable to further include first musical piece reproduction equipment that is able to acoustically reproduce an acoustic signal included in the musical pieces, and a storage section that stores the one or the plurality of particular musical pieces. It is preferable that the first musical piece reproduction equipment is controlled by the control unit.

According to the configuration, there is no need for the user to separately prepare musical piece reproduction equipment such as a portable audio player.

In addition, it is preferable to further include connection means for connecting second musical piece reproduction equipment which is able to acoustically reproduce an acoustic signal included in the musical pieces, by wire or radio. It is preferable that the one or the plurality of particular musical pieces are stored in the second musical piece reproduction equipment.

According to the configuration, even if the massage machine is not provided with the musical piece reproduction equipment, it is possible to utilize the musical piece reproduction equipment such as the portable audio player possessed by the user.

In addition, it is preferable to further include connection means for connecting second musical piece reproduction equipment which is able to acoustically reproduce an acoustic signal included in the musical pieces, by wire or radio. It is preferable that a second massage course in which the massage portion is operated based on a variation of an acoustic signal reproduced by the second musical piece reproduction equipment is stored. It is preferable that the control unit is able to execute the second massage course.

According to the configuration, it is possible to perform a massage tuned to the tempo of an arbitrary musical piece stored in the portable audio player or the like possessed by the user.

In addition, it is preferable to further include a storage section that is able to store a musical piece desired by the user. It is preferable that a second massage course in which the massage portion is operated based on a variation of an acoustic signal included in the musical pieces stored in the storage section is stored. It is preferable that the control unit is able to execute the second massage course.

According to the configuration, it is possible to perform a massage tuned to the tempo of an arbitrary musical piece possessed by the user.

In addition, it is preferable that in the second massage course, the control unit compares levels between a threshold value set in advance and the acoustic signal reproduced by the second musical piece reproduction equipment, so as to change an operational condition of the massage portion.

According to the configuration, it is possible to change the operational condition of the massage portion in accordance with the acoustic signal.

In addition, it is preferable to further include a selection section that selects any of the first massage course and the second massage course.

According to the configuration, it is possible to select any of the first massage course which is favorably tuned to the tempo of the musical piece, and the second massage course which is able to correspond to the tempo of an arbitrary musical piece.

In addition, it is preferable to further include a first switching section that switches a state to any one of a silent state where an acoustic signal reproduced by the first or second musical piece reproduction equipment is prohibited from being acoustically reproduced and a reproduction state where the acoustic signal is acoustically reproduced, while being in a state where the massage portion is continuously operated.

According to the configuration, it is possible to make the user feel the acoustic signal as a massage operation performed by the massage portion, instead of a sound.

In addition, it is preferable to further include a second switching section that switches a state to any one of an operation state where the massage portion is operated and a halt state where the massage portion is halted, while being in a state where an acoustic signal is continuously and acoustically reproduced by the first or second musical piece reproduction equipment.

According to the configuration, while making the user acoustically feel the acoustic signal, it is possible to select whether or not to operate the massage portion.

In addition, it is preferable to further include sound emission means for acoustically outputting an acoustic signal reproduced by the first or second musical piece reproduction equipment.

According to the configuration, it is possible to make the user acoustically feel the acoustic signal.

### ADVANTAGE OF THE INVENTION

According to the present invention, it is possible to perform a massage tuned to the tempo of the musical piece.

### BRIEF DESCRIPTION OF THE DRAWINGS

- Fig. 1: is a side view of a massage machine according to an embodiment of the present invention.
- Fig. 2: is a front view of a massage unit.
- Fig. 3: is a block diagram of the massage machine.
- Fig. 4: is a view describing a correspondence relationship between the tempo of a musical piece and the lifting/lowering speed of the massage unit. Fig. 4(a) illustrates a variation of the tempo of the musical piece, and Fig. 4(b) illustrates a variation of the lifting/lowering speed of the massage unit.
- Fig. 5: is a view describing a variation of an acoustic signal and an operation of each massage portion in a first massage course.
- Fig. 6: is a view illustrating a touch panel. Fig. 6(a) illustrates a selection screen for a massage course, Fig. 6(b) illustrates a selection screen for a musical piece, and Fig. 6(c) illustrates a selection screen for starting a second massage course.
- Fig. 7: is a view describing an operation of each massage portion based on the acoustic signal in the second massage course.
- Fig. 8: is a view describing another operation of each massage portion based on the acoustic signal in the second massage course.

### Overall Configuration of Massage Machine

Hereinafter, the overall configuration of a massage machine 1 according to an embodiment of the present invention will be described.

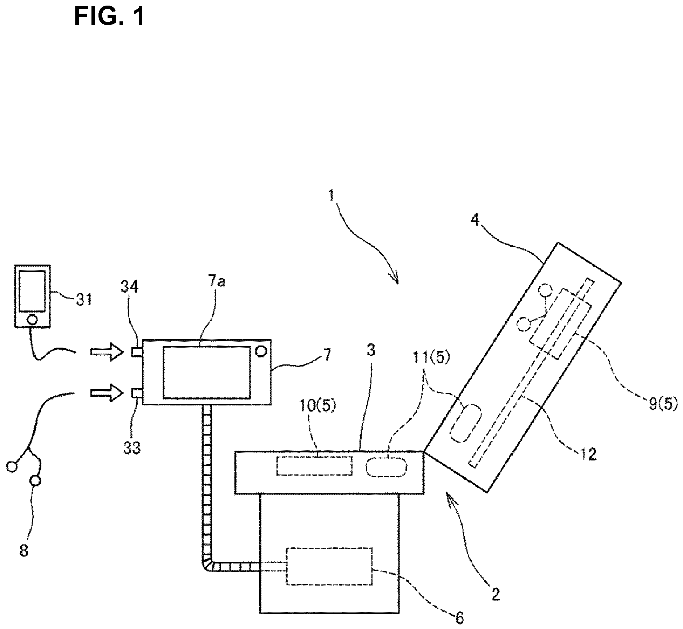

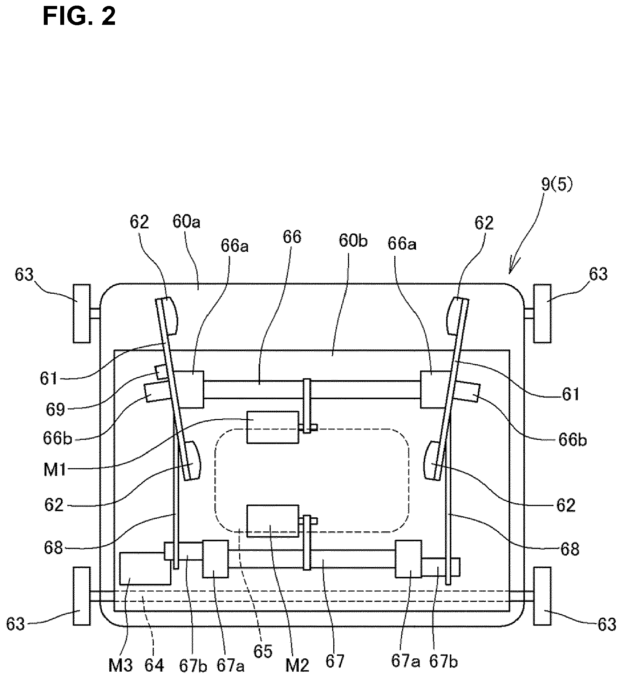

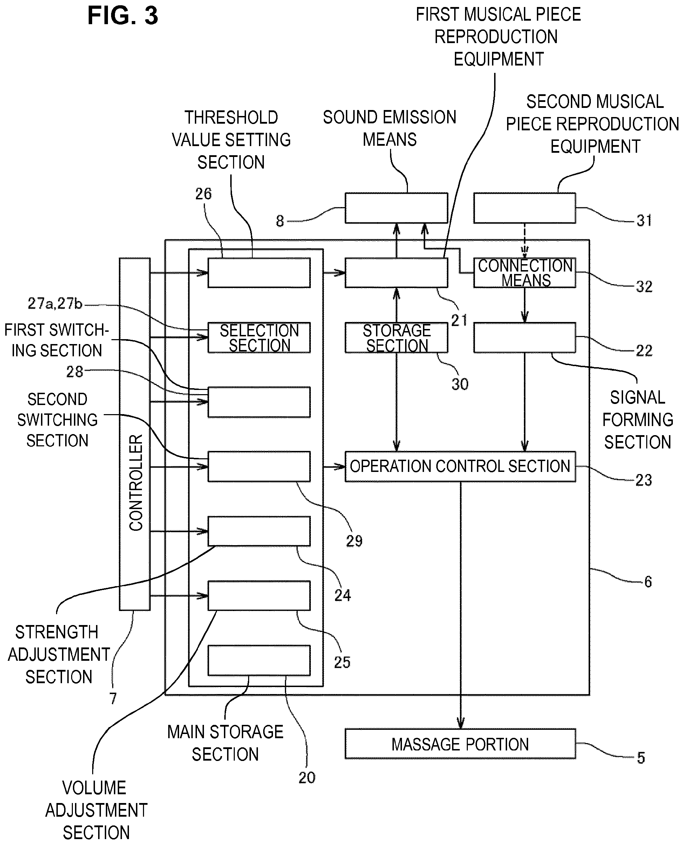

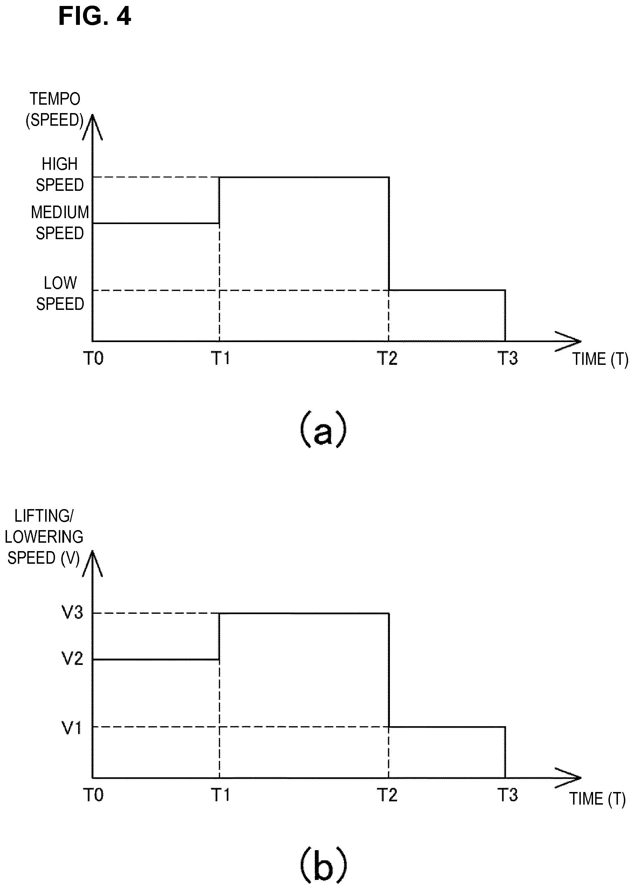

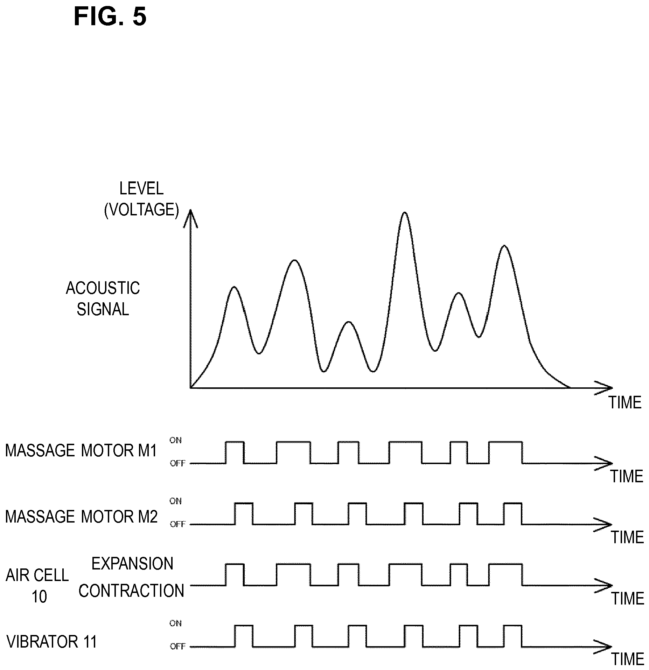

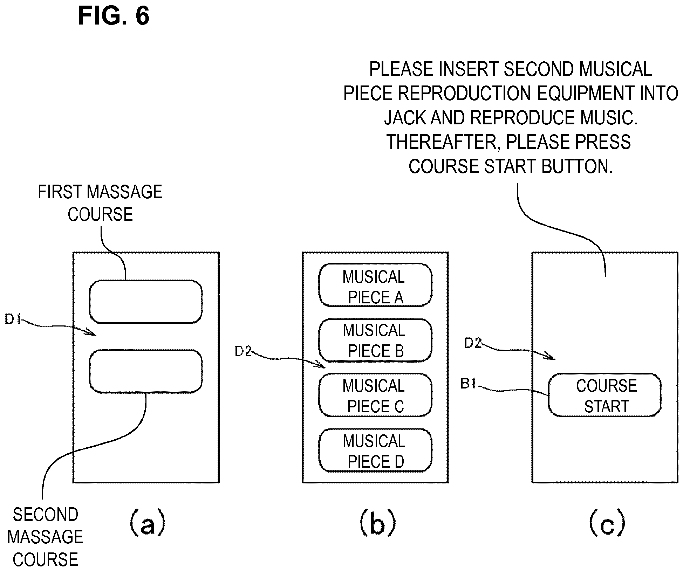

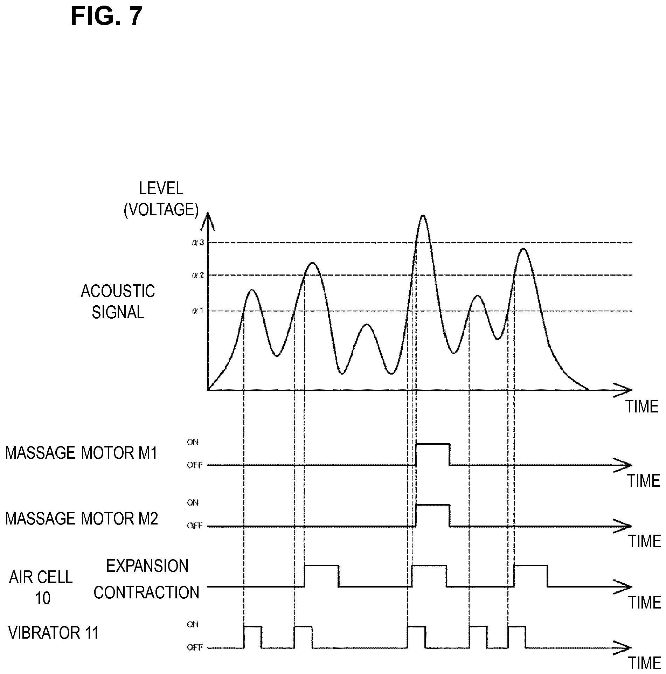

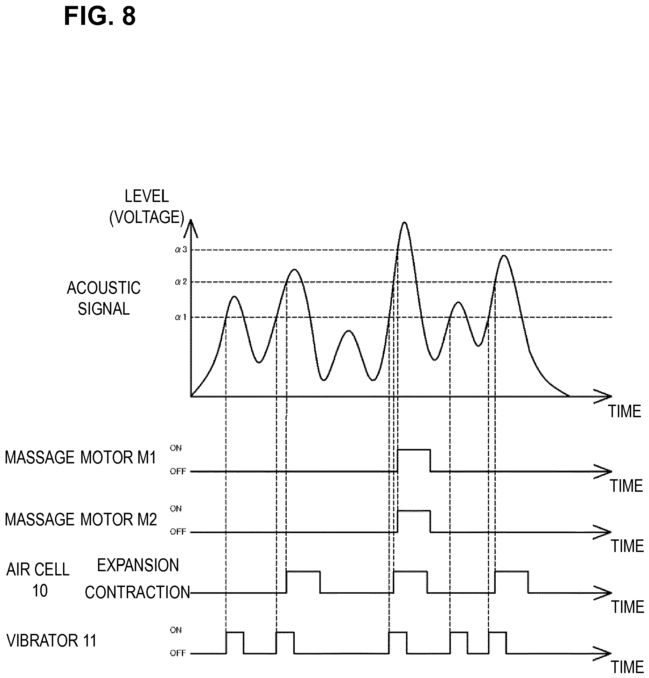

Fig. 1 is a side view of the massage machine 1 according to the embodiment of the present invention. Fig. 2 is a front view of a massage unit 9. Fig. 3 is a block diagram of the massage machine 1. Fig. 4 is a view describing a correspondence relationship between the tempo of a musical piece A and the lifting/lowering speed of the massage unit 9. Fig. 4(a) illustrates a variation of the tempo of the musical piece A, and Fig. 4(b) illustrates a variation of the lifting/lowering speed of the massage unit 9. Fig. 5 is a view describing a variation of an acoustic signal and an operation of each massage portion 5 in a first massage course. Fig. 6 is a view illustrating a touch panel 7a. Fig. 6(a) illustrates a selection screen for a massage course, Fig. 6(b) illustrates a selection screen for a musical piece, and Fig. 6(c) illustrates a selection screen for starting a second massage course. Fig. 7 is a view describing an operation of each massage portion 5 based on the acoustic signal in the second massage course. Fig. 8 is a view describing another operation of each massage portion 5 based on the acoustic signal in the second massage course.

As illustrated in Figs. 1 to 3, the massage machine 1 according to the embodiment of the present invention has a seat portion 3 in which at least a user takes a seat, and a backrest portion 4 which is provided at the back of the seat portion 3 and on which the user leans. The seat portion 3 and the backrest portion 4 configure a main body portion 2. In addition, the massage machine 1 has the massage portion 5 which performs a massage with respect to the user, a control unit 6 which controls various types of operations of the massage machine 1, a controller 7 through which the user performs various types of operations, and sound emission means 8 for acoustically outputting an acoustic signal reproduced by first musical piece reproduction equipment 21 or second musical piece reproduction equipment 31 (will be described later). It is preferable that the controller 7 is provided with the touch panel 7a operated by the user with a fingertip. The main body portion 2 may have a footrest provided in a front portion of the seat portion 3, and armrest portions provided on both the sides of the seat portion 3. The footrest or the armrest portions may be provided with the massage portion 5.

As the massage portion 5, there is provided at least one of the massage unit 9 which has treatment members 62 making a pair on the right and left and being driven by motors M1 to M3, an air cell 10 which expands and contracts in response to air supply/exhaust, and a vibrator 11 which is driven by a motor. In the present embodiment, the massage unit 9 and the vibrator 11 are provided in the backrest portion 4, and the air cell 10 and the vibrator 11 are provided in the seat portion 3. However, disposition of each massage portion 5 is not limited thereto. The air cell 10 can press the user through air supply/exhaust. The vibrator 11 can apply vibration to the user by rotating an eccentric weight portion.

### Configuration of Massage Unit

As illustrated in Fig. 2, the backrest portion 4 is provided with the massage unit 9 which massages the upper half of the body of the user from the back (back surface). The massage unit 9 is configured to include arms 61 making a pair on the right and left, and the treatment members 62 provided in both upper and lower end portions in each arm 61. The massage unit 9 can perform a kneading massage in which the treatment members 62 on the right and left approach each other and are separated from each other in response to driving of the massage motor M1, and a patting massage in which the treatment members 62 on the right and left alternately advance and retreat with respect to the user side in response to driving of the massage motor M2. In addition, the massage unit 9 moves upward or downward along the height direction in response to driving of the lifting/lowering motor M3. Accordingly, the massage unit 9 can change the position with respect to the body or can perform a rolling massage. In the backrest portion 4, there are provided guide rails 12 making a pair on the right and left and extending in the height direction (refer to Fig. 1), and the massage unit 9 moves along the guide rails 12. Since the massage unit 9 can move in the height direction, the user can be massaged by the treatment members 62 from the neck to the waist.

As illustrated in Fig. 2, the massage unit 9 has a base frame 60a and a movable frame 60b which is supported by the base frame 60a. The base frame 60a has guide rollers 63 which are fitted to both the right and left sides of the guide rails 12. The base frame 60a can be moved along the height direction by a lifting/lowering mechanism (not illustrated) formed with rack and pinion, or the like. The movable frame 60b is supported by the base frame 60a via an oscillation axis 64 in the lateral direction. Between the base frame 60a and the movable frame 60b, there is provided an advancing/retreating drive portion 65 formed with an air cell or the like. In response to driving of the advancing/retreating drive portion 65, the movable frame 60b can advance and retreat with respect to the user while having the oscillation axis 64 as the center. It is not compulsory to have the structure in which the movable frame 60b advances and retreats. A structure in which the arms 61 are provided with the advancing/retreating drive portions 65 and only the arms 61 advance and retreat may be adopted.

The arms 61 are interlocked with a kneading shaft 66 and a patting shaft 67 extending in the lateral direction. On both the right and left sides of the kneading shaft 66, there are respectively provided bevel cams 66a having bevel shaft portions 66b. The arms 61 are respectively attached to the bevel cams 66a. The bevel shaft portions 66b on the right and left are beveled with respect to the shaft center of the kneading shaft 66 so as to form a substantially inverted V-shape in a front view. On both the right and left sides of the patting shaft 67, there are respectively provided eccentric cams 67a having eccentric shaft portions 67b which are eccentric with respect to the shaft center of the patting shaft 67. The arms 61 are respectively attached to the eccentric cams 67a via connecting rods 68. In the eccentric shaft portions 67b on the right and left, the phases with respect to the shaft center of the patting shaft 67 are different from each other. Specifically, the phases are different from each other as much as 180 degrees. The kneading shaft 66 and the patting shaft 67 respectively rotate in response to driving of the massage motors M1 and M2. The treatment members 62 performs a kneading massage in response to rotation of the kneading shaft 66 and performs a patting massage in response to rotation of the patting shaft 67. The arms 61 provided with the treatment members 62, the kneading shaft 66, and the patting shaft 67 are supported by the movable frame 60b. Therefore, the treatment members 62 can advance and retreat with respect to the user via movement of the movable frame 60b.

The arms 61 oscillate freely in the forward/backward direction, and the upper treatment members 62 are biased by biasing means (not illustrated) formed with a spring or the like so as to protrude forward. In addition, the massage unit 9 has a sensor 69 which detects body information of the user. The sensor 69 can acquire the body information by detecting that the arms 61 are at predetermined oscillation positions. Specifically, in a process of lifting the massage unit 9 along the height direction, when the upper treatment members 62 reach upper portions on the shoulder, loads acting on the treatment members 62 are cancelled, and the arms 61 oscillate forward due to action of the biasing means (not illustrated), thereby being at predetermined oscillation positions. The sensor 69 detects that the arms 61 are at predetermined oscillation positions. The sensor 69 detects the position of the shoulder based on the vertical position of the massage unit 9 at that time. Based on the position of the shoulder, positions of other sites (neck, back, waist, and the like) are obtained through calculation. The detected body information is stored in a storage section 30 of the control unit 6. The user may directly input the body information. In this case, for example, the body information can be input by operating the controller 7, and the input body information can be stored in the storage section 30 of the control unit 6.

### Configuration of Control Unit

The control unit 6 is formed with a programmable microcomputer having a CPU (not illustrated) and a main storage section 20. The main storage section 20 stores a program for executing each of predetermined functions. As functioning sections executed by the program, the control unit 6 has the first musical piece reproduction equipment 21, a signal forming section 22, an operation control section 23, a strength adjustment section 24, a volume adjustment section 25, a threshold value setting section 26, a selection section 27a, a selection section 27b, a first switching section 28, and a second switching section 29. The functioning sections will be described later. In addition, the control unit 6 has the storage section 30 which stores one or a plurality of massage courses, and one or a plurality of particular musical pieces. In addition, the control unit 6 has connection means 32 for connecting the second musical piece reproduction equipment 31 independent from the sound emission means 8 and the massage machine 1. The controller 7 is electrically connected to the strength adjustment section 24, the volume adjustment section 25, the threshold value setting section 26, the selection section 27a, the selection section 27b, the first switching section 28, and the second switching section 29.

The connection means 32 included in the control unit 6 is electrically connected to a jack 33 provided in the controller 7. When a plug included in the sound emission means 8 formed with an earphone or the like is inserted into this jack 33, the sound emission means 8 can acoustically output an acoustic signal reproduced by the first musical piece reproduction equipment 21. In addition, a plug included in the second musical piece reproduction equipment 31 independent from the massage machine 1 can be inserted into a jack 34. It is preferable that the second musical piece reproduction equipment is a portable audio player, a smart phone, or the like possessed by the user. In this case, the sound emission means 8 can acoustically output an acoustic signal reproduced by the second musical piece reproduction equipment 31. The connection means 32 may be radio communication means for performing short-range radio communication by infrared rays or the like. In addition, the sound emission means 8 may be a speaker attached to the backrest portion 4 or the like.

In the storage section 30, the particular musical piece is housed in advance by a manufacturer of the massage machine 1 of the present invention. In the present embodiment, a plurality of musical pieces A to D are stored as the particular musical pieces. However, one musical piece may be stored. In the storage section 30, it is preferable to store the musical pieces A and B of a slow tempo introducing the user to a relaxed state, for example, and the musical pieces C and D of a quick tempo introducing the user to an activated state, for example. In addition, the storage section 30 stores one or a plurality of the first massage courses in which the massage portion 5 performs an operation determined in advance in a manner corresponding to the one or the plurality of particular musical pieces A to D, and the second massage course in which the massage portion 5 is operated based on a variation of an acoustic signal reproduced by the second musical piece reproduction equipment 31.

In the present embodiment, the plurality of particular musical pieces A to D are stored in the storage section 30. In addition, first massage courses a to d respectively corresponding to the musical pieces A to D are stored in the storage section 30. That is, the massage courses are designed in advance such that the first massage course a is tuned to the tempo of the musical piece A, the first massage course b is tuned to the tempo of the musical piece B, the first massage course c is tuned to the tempo of the musical piece C, and the first massage course d is tuned to the tempo of the musical piece D.

As a matter of course, in the acoustic signal, there exist the tempo (speed) and the degree of strength of the sound depending on the musical piece. Since the acoustic signal is a digital signal, the degree of strength of the sound is expressed in the level (voltage) of the acoustic signal. In the first massage courses a to d, in order to be tuned to the tempo of the corresponding musical pieces A to D, the operation control section 23 varies at least any one of the speed of kneading, patting, or lifting/lowering of the massage unit 9; the interval switching between expansion and contraction of the air cell 10; and the speed of vibration of the vibrator 11 in time series. Hereinafter, the correspondence relationship between the tempo of the musical piece and the operation speed of the massage portion 5 in the first massage course will be described based on Fig. 4. The variation of the operation speed of the massage portion 5 will be described by exemplifying the variation of the lifting/lowering speed of the massage unit 9.

Fig. 4(a) representatively illustrates the variation of the tempo of the musical piece A among the plurality of musical pieces A to D stored in the storage section 30. The tempo of the musical piece A varies in time series. Time section T0 to T1 indicates the tempo of "medium speed", time section T1 to T2 indicates the tempo of "high speed", and time section T2 to T3 indicates the tempo of "low speed". Fig. 4(b) representatively illustrates the variation of the lifting/lowering speed of the massage unit 9 in the first massage course a, among a plurality of the first massage courses a to d stored in the storage section 30. The lifting/lowering speed of the massage unit 9 in the first massage course a varies in time series. Specifically, the lifting/lowering speed of the massage unit 9 indicates "speed V2" in the time section T0 to T1, indicates "speed V3" in the time section T1 to T2, and indicates "speed V1" in the time section T2 to T3, thereby establishing V3>V2>V1.

In this manner, in the first massage course, when the tempo of the musical piece becomes quicker, the operation speed of the massage portion 5 is set to be high, and when the tempo of the musical piece becomes slower, the operation speed of the massage portion 5 is set to be low. The variation of the operation speed of the massage portion 5 is described by exemplifying the lifting/lowering speed of the massage unit 9. However, it is preferable to similarly set other operations of the massage unit 9, or operations of a different massage portion 5. In other words, it is preferable that when the tempo of the musical piece becomes quicker, the speed of kneading or patting of the massage unit 9 is set to be high, when the tempo of the musical piece becomes quicker, the interval of switching between expansion and contraction of the air cell 10 is set to be short, and when the tempo of the musical piece becomes quicker, the speed of vibration of the vibrator 11 is set to be high.

In addition, in order to be tuned to the waveform of the acoustic signal of the corresponding musical piece, or the strength of the sound of the corresponding musical piece, the operation control section 23 varies driving of at least one among the massage portions 5 in time series. Hereinafter, the correspondence relationship between the acoustic signal and the operation of each massage portion 5 in the first massage course will be exemplified based on Fig. 5. As illustrated in Fig. 5, driving of the massage motor M1 included in the massage unit 9 is set to be tuned to the waveform of the acoustic signal. That is, when the width between the peaks of the waveform becomes greater, the continuous drive time of the massage motor M1 is lengthened, and when the width between the peaks of the waveform becomes smaller, the continuous drive time of the massage motor M1 is shortened. The massage motor M2 included in the massage unit 9 is set to be driven for a certain period of time corresponding to the peaks of the acoustic signal. Driving of the air cell 10 is set to be tuned to the waveform of the acoustic signal. That is, when the width between the peaks of the waveform becomes greater, the period of time for continuous air supply with respect to the air cell 10 is lengthened, and when the width between the peaks of the waveform becomes smaller, the period of time for continuous air supply with respect to the air cell 10 is shortened. The vibrator 11 is set to be driven for a certain period of time corresponding to the peaks of the acoustic signal.

In this manner, the massage courses tuned to the tempo of the particular musical pieces designated by the manufacturer, the waveform of the acoustic signal, or the strength of the sound are designed in advance. Therefore, there is no need for the operation of the massage portion 5 to successively correspond to the variation of the acoustic signal, and it is possible to perform a massage intended by the manufacturer so as to be tuned to the tempo of the musical piece, the waveform of the acoustic signal, or the strength of the sound.

The strength adjustment section 24 changes massage force of each massage portion 5 while the first massage course is executed. The massage force of the massage unit 9 is changed by changing the forward/backward position of the movable frame 60b. In addition, the massage force of the massage unit 9 may be changed by changing the speed of kneading and/or patting. Generally, when the speed of kneading or patting becomes higher, the user can have strong feeling. The air cell 10 changes the massage force by changing the period of time for continuous air supply with respect to a predetermined air cell 10. The vibrator 11 changes the massage force by changing the drive speed. The strength adjustment section 24 may be configured to collectively change the massage force of the massage portions 5 or may be configured to individually change the massage force thereof. In addition, the volume adjustment section 25 changes the level of the acoustic signal reproduced by the first musical piece reproduction equipment 21 or the second musical piece reproduction equipment 31. Therefore, the volume of a sound output by the sound emission means 8 is changed.

While the first massage course is executed, in a state where each massage portion 5 is continuously operated, the first switching section 28 switches a state to any one of a silent state where an acoustic signal reproduced by the first musical piece reproduction equipment 21 or the second musical piece reproduction equipment 31 is prohibited from being acoustically reproduced and a reproduction state where the acoustic signal is acoustically reproduced. In a case of the silent state, it is possible to make the user feel the acoustic signal as a massage operation of the massage portion 5, instead of a sound. Meanwhile, in a case of the reproduction state, it is possible to make the user feel the acoustic signal as a sound and a massage operation synchronized with the sound of the massage portion 5.

While the first massage course or the second massage course (will be described later) is executed, in a state where the acoustic signal is continuously and acoustically reproduced by the first musical piece reproduction equipment 21 or the second musical piece reproduction equipment 31, the second switching section 29 switches a state to any one of an operation state where the massage portion 5 is operated and a halt state where the massage portion 5 is halted. In other words, while making the user acoustically feel the acoustic signal, it is possible to select whether or not to operate the massage portion 5. In this specification, the state where the operation control section 23 controls the operation of at least one of the massage portions 5 so as to perform a massage operation will be referred to as "operation state".

When the user operates the controller 7, the selection section 27a included in the control unit 6 selects the corresponding musical piece. For example, when the user selects "musical piece A" as a desired musical piece from the plurality of musical pieces A to D stored in the storage section 30, the selection section 27a selects the acoustic signal of this "musical piece A" as a target signal and causes the first musical piece reproduction equipment 21 to perform reproduction. Simultaneously, the operation control section 23 executes the first massage course a corresponding to the musical piece A. When the selection section 27a selects the corresponding musical piece, after acquiring of the body information by the sensor 69 is completed, reproduction of the acoustic signal and execution of the first massage course may start.

The signal forming section 22 forms an operational control signal for operating the massage portion 5, based on an acoustic signal input from the second musical piece reproduction equipment 31. The operational control signal formed by the signal forming section 22 based on the acoustic signal is a signal synchronized with the acoustic signal. In other words, the signal forming section 22 forms an operational control signal for switching the massage operation when a predetermined variation appears in the level of the acoustic signal. However, the timing for the variation of the level of the acoustic signal and the timing for switching the massage operation are simultaneous.

The main storage section 20 of the control unit 6 stores threshold values α1 to α3 set in advance. The threshold values α1 to α3 are voltage values and are compared with the level (voltage) of the acoustic signal. In the present embodiment, a plurality of the threshold values α1 to α3 are stored. The signal forming section 22 has a function of comparing the threshold values α1 to α3 and the level of the acoustic signal. When the threshold values α1 to α3 and the level of the acoustic signal reproduced by the second musical piece reproduction equipment 31 are compared with each other, the operation control section 23 changes the operational condition of each massage portion 5.

In the second massage course, a lifting/lowering operation of the massage unit 9 is set in advance. However, other operations are not set. In other words, in the second massage course, in a process in which the massage unit 9 performs a predetermined lifting/lowering operation for a predetermined period of time, based on the variation of the acoustic signal reproduced by the second musical piece reproduction equipment 31, kneading or patting of the massage unit 9, and the operational conditions of the air cell 10 and the vibrator 11 are changed.

Hereinafter, a method of changing the operational condition of each massage portion 5 will be exemplified. For example, as illustrated in Fig. 7, the type of the massage portion 5 driven in accordance with the level of the acoustic signal can be changed. In this case, it is preferable to perform controlling as follows. When the acoustic signal does not exceed any of the threshold values α1 to α3, none of the massage portions 5 is driven. When the acoustic signal exceeds the threshold value α1 and does not exceed the threshold value α2 within a predetermined period of time, the vibrator 11 is driven (vibrated) for a predetermined period of time. When the acoustic signal exceeds the threshold value α1, exceeds the threshold value α2 within a predetermined period of time, and does not exceed the threshold value α3 within a predetermined period of time, the vibrator 11 is driven (vibrated) for a predetermined period of time and the air cell 10 is driven (caused to expand) for a predetermined period of time. When the acoustic signal exceeds the threshold value α1, exceeds the threshold value α2 within a predetermined period of time, and exceeds the threshold value α3 within a predetermined period of time, the vibrator 11 is driven (vibrated) for a predetermined period of time, the air cell 10 is driven (caused to expand) for a predetermined period of time, and the massage unit 9 is caused to perform kneading and/or patting for a predetermined period of time.

In addition, as illustrated in Fig. 8, the massage force of the massage portion 5 can be changed in accordance with the level of the acoustic signal. In this case, it is preferable to perform controlling as follows. When the acoustic signal does not exceed any of the threshold values α1 to α3, none of the massage portions 5 is driven. When the acoustic signal exceeds the threshold value α1 and does not exceed the threshold value α2 within a predetermined period of time, the vibrator 11 is driven (vibrated) for a predetermined period of time at a speed v1, air is supplied to the air cell 10 for a predetermined period of time t1, and the massage unit 9 is caused to perform kneading and/or patting for a predetermined period of time at the speed v1. When the acoustic signal exceeds the threshold value α1, exceeds the threshold value α2 within a predetermined period of time, and does not exceed the threshold value α3 within a predetermined period of time, while varying from the speed v1 to a speed v2, the vibrator 11 is driven (vibrated) for a predetermined period of time, air is supplied to the air cell 10 for a predetermined period of time t2, and while varying from the speed v1 to the speed v2, the massage unit 9 is caused to perform kneading and/or patting for a predetermined period of time. When the acoustic signal exceeds the threshold value α1, exceeds the threshold value α2 within a predetermined period of time, and exceeds the threshold value α3 within a predetermined period of time, while varying from the speed v1 to a speed v3, the vibrator 11 is driven (vibrated) for a predetermined period of time, air is supplied to the air cell 10 for a predetermined period of time t3, and while varying from the speed v1 to the speed v3, the massage unit 9 is caused to perform kneading and/or patting for a predetermined period of time. It is preferable to establish v3>v2>v1 and t3>t2>t1.

The threshold value setting section 26 changes the levels of the threshold values α1 to α3. The volume adjustment section 25 changes the level of the acoustic signal reproduced by the second musical piece reproduction equipment 31. When the levels of the threshold values α1 to α3 are raised or the level of the acoustic signal is lowered, the number of times each massage portion 5 is in the operation state is reduced. In other words, the number of times the massage unit 9 performs kneading and/or patting is reduced, the number of cycles the air cell 10 expands and contracts is reduced, and the number of cycles the vibrator 11 is vibrated and halted is reduced. When the levels of the threshold values α1 to α3 are lowered or the level of the acoustic signal is raised, the number of times each massage portion 5 is in the operation state increases. In other words, the number of times the massage unit 9 performs kneading and/or patting increases, the number of cycles the air cell 10 expands and contracts increases, and the number of cycles the vibrator 11 is vibrated and halted increases. The threshold value setting section 26 may be able to collectively adjust the levels of the plurality of threshold values α1 to α3 or may be able to individually adjust the levels thereof.

Hereinafter, a method of selecting the massage course will be described based on Fig. 6.

When the user operates the controller 7, the selection section 27b included in the control unit 6 selects the corresponding massage course. In the touch panel 7a of the controller 7, as an initial screen, it is preferable that a selection screen D1 for the massage course illustrated in Fig. 6(a) is displayed. First, the user selects any one of the first massage course and the second massage course through the selection screen D1 for the massage course. In a case where the first massage course is selected through the screen D1, the screen of the touch panel 7a shifts to a selection screen D2 for the musical piece illustrated in Fig. 6(b). Subsequently, the user selects a desired musical piece from the musical pieces A to D through the selection screen D2 for the musical piece. When the musical piece is selected, the control unit 6 reproduces the selected musical piece and executes the corresponding first massage course.

Meanwhile, in a case where the second massage course is selected through the selection screen D1 for the massage course illustrated in Fig. 6(a), the screen of the touch panel 7a shifts to a selection screen D3 for starting the second massage course illustrated in Fig. 6(c). In the screen D3, guidance urging the user to connect the massage machine 1 and the second musical piece reproduction equipment 31 is displayed, such as "Please insert the second musical piece reproduction equipment into the jack and reproduce music. Thereafter, please press the course start button", and a course start button B1 of the second massage course is displayed. When the course start button B1 is pressed, the control unit 6 executes the second massage course.

In addition, the massage machine 1 of the present invention is not limited to the illustrated forms, and other forms may be adopted within the scope of the invention.

For example, similar to the first musical piece reproduction equipment 21, the second musical piece reproduction equipment 31 may be built in the massage machine 1. In addition, the massage machine 1 may be configured such that the user can store a favorite musical piece in the storage section 30 of the control unit 6 via a record medium or a network. The acoustic signal of the musical piece can be utilized as an acoustic signal for executing the second massage course. In addition, the one or the plurality of musical pieces A to D prepared by the manufacturer may be stored in the second musical piece reproduction equipment 31 formed with a portable audio player, a smart phone, or the like. In this case, the musical pieces A to D may be downloaded to the second musical piece reproduction equipment 31 via a network by wire or radio. Otherwise, the musical pieces A to D may be stored in the second musical piece reproduction equipment 31 via a record medium formed with a USB memory or the like.

### INDUSTRIAL APPLICABILITY

The present invention can be applied to a massage machine which is able to perform a massage tuned to the tempo of a musical piece.

### LIST OF REFERENCE NUMERALS

- 1: MASSAGE MACHINE
- 5: MASSAGE PORTION
- 6: CONTROL UNIT
- 8: SOUND EMISSION MEANS
- 21: FIRST MUSICAL PIECE REPRODUCTION EQUIPMENT
- 27a: SELECTION SECTION
- 27b: SELECTION SECTION
- 28: FIRST SWITCHING SECTION
- 29: SECOND SWITCHING SECTION
- 30: STORAGE SECTION
- 31: SECOND MUSICAL PIECE REPRODUCTION EQUIPMENT
- 32: CONNECTION MEANS
- α1 TO α3: THRESHOLD VALUE

## Claims

1. A massage machine comprising:
a massage portion that performs a massage with respect to a user; and
a control unit that controls an operation of the massage portion,
wherein one or a plurality of first massage courses in which the massage portion performs an operation determined in advance in a manner corresponding to one or a plurality of particular musical pieces are stored, and
wherein the control unit is able to execute the first massage course.

2. The massage machine according to Claim 1, further comprising:
first musical piece reproduction equipment that is able to acoustically reproduce an acoustic signal included in the musical pieces; and
a storage section that stores the one or the plurality of particular musical pieces,
wherein the first musical piece reproduction equipment is controlled by the control unit.

3. The massage machine according to Claim 1 or 2, further comprising:
connection means for connecting second musical piece reproduction equipment which is able to acoustically reproduce an acoustic signal included in the musical pieces, by wire or radio,
wherein the one or the plurality of particular musical pieces are stored in the second musical piece reproduction equipment.

4. The massage machine according to any one of Claims 1 to 3, further comprising:
connection means for connecting second musical piece reproduction equipment which is able to acoustically reproduce an acoustic signal included in the musical pieces, by wire or radio,
wherein a second massage course in which the massage portion is operated based on a variation of an acoustic signal reproduced by the second musical piece reproduction equipment is stored, and
wherein the control unit is able to execute the second massage course.

5. The massage machine according to any one of Claims 1 to 4, further comprising:
a storage section that is able to store a musical piece desired by the user,
wherein a second massage course in which the massage portion is operated based on a variation of an acoustic signal included in the musical pieces stored in the storage section is stored, and
wherein the control unit is able to execute the second massage course.

6. The massage machine according to Claim 4 or 5,
wherein in the second massage course, the control unit compares levels between a threshold value set in advance and the acoustic signal reproduced by the second musical piece reproduction equipment or an acoustic signal included in the musical piece desired by the user, so as to change an operational condition of the massage portion.

7. The massage machine according to any one of Claims 4 to 6, further comprising:
a selection section that selects any of the first massage course and the second massage course.

8. The massage machine according to any one of Claims 2 to 7, further comprising:
a first switching section that switches a state to any one of a silent state where an acoustic signal reproduced by the first or second musical piece reproduction equipment is prohibited from being acoustically reproduced and a reproduction state where the acoustic signal is acoustically reproduced, while being in a state where the massage portion is continuously operated.

9. The massage machine according to any one of Claims 2 to 8, further comprising:
a second switching section that switches a state to any one of an operation state where the massage portion is operated and a halt state where the massage portion is halted, while being in a state where an acoustic signal is continuously and acoustically reproduced by the first or second musical piece reproduction equipment.

10. The massage machine according to any one of Claims 2 to 9, further comprising:
sound emission means for acoustically outputting an acoustic signal reproduced by the first or second musical piece reproduction equipment.
